Europäisches Patentamt

European Patent Office

Office européen des brevets

(11) Publication number: **0 205 811**
**A1**

# (12) EUROPEAN PATENT APPLICATION

(21) Application number: **86105508.5**

(22) Date of filing: **21.04.86**

(51) Int. Cl.⁴: **A 61 B 17/00**

(30) Priority: **18.06.85 AU 1081/85**

(43) Date of publication of application: **30.12.86**
**Bulletin 86/52**

(84) Designated Contracting States: **DE FR GB**

(71) Applicant: **Lee, Wilson Kam Chor, Dr., 14 Thornber Street, Unley Park South Australia 5061 (AU)**

(72) Inventor: **Lee, Wilson Kam Chor, Dr., 14 Thornber Street, Unley Park South Australia 5061 (AU)**

(74) Representative: **VOSSIUS & PARTNER, Siebertstrasse 4 P.O. Box 86 07 67, D-8000 München 86 (DE)**

(54) **Surgical needle.**

(57) A surgical needle (10; 50) characterized by having two sharpened ends (20, 25; 60, 65) together with a centrally or eccentrically placed eye (30; 70) or attached suture, in which the needle may be either straight or curved. Embodiments suitable for use in orthopaedic operations are described.

# VOSSIUS & PARTNER

0205811

PATENTANWÄLTE

EUROPEAN PATENT ATTORNEYS

Dr. VOLKER VOSSIUS, Dipl.-Chem.
DOROTHEA VOSSIUS, Dipl.-Chem.
Dr. PAUL TAUCHNER, Dipl.-Chem.
Dr. DIETER HEUNEMANN, Dipl.-Phys.
Dr. PETER RAUH, Dipl.-Chem.
Dr. GERHARD HERMANN, Dipl.-Phys.

SIEBERTSTRASSE 4
P.O. BOX 860767
8000 MÜNCHEN 86
PHONE: (089) 474075
CABLE: BENZOLPATENT MÜNCHEN
TELEX: 529453 VOPAT D
TELEFAX: (089) 472001 (GR. II + III)

Our Ref.: U 533 EP
Case: VS:X346
Dr. Wilson Kam Chor LEE
Australia

April 21, 1986

## SURGICAL NEEDLE

This invention relates to improvements in or relating to surgical needles.

Advances in surgery depend in part on the availability to surgeons of instruments suitable for use in confined spaces.

It is an object of the present invention to provide a needle for suturing which enables surgical sutures to be inserted in a structure within a confined anatomical site, without the necessity for knotting the sutures externally to that site.

The present invention is particularly useful in the repair of lesions of the meniscus of the knee joint, and will accordingly be described in more detail in relation to that field of use. However, its usefulness in other fields will be apparent and it will be understood that the invention is not limited to use only in this context. For example, applications in the fields of urology and laparoscopic abdominal surgery are contemplated.

Preferred embodiments of the invention will be further illustrated by the following non-limiting examples, with reference to the accompanying figures, in which:

Figure 1 represents a diagrammatic view of a transverse section through the human knee joint as seen from above;

Figure 2 represents perspective views of the preferred embodiments of the invention.

Figure 3 represents diagrammatically the principle of operation of the needle of the invention;

Figure 4 represents diagrammatically the use of the straight needle of the invention for repair of a meniscal tear; and

Figure 5 represents diagrammatically the use of the curved needle of the invention for repair of a tendon.

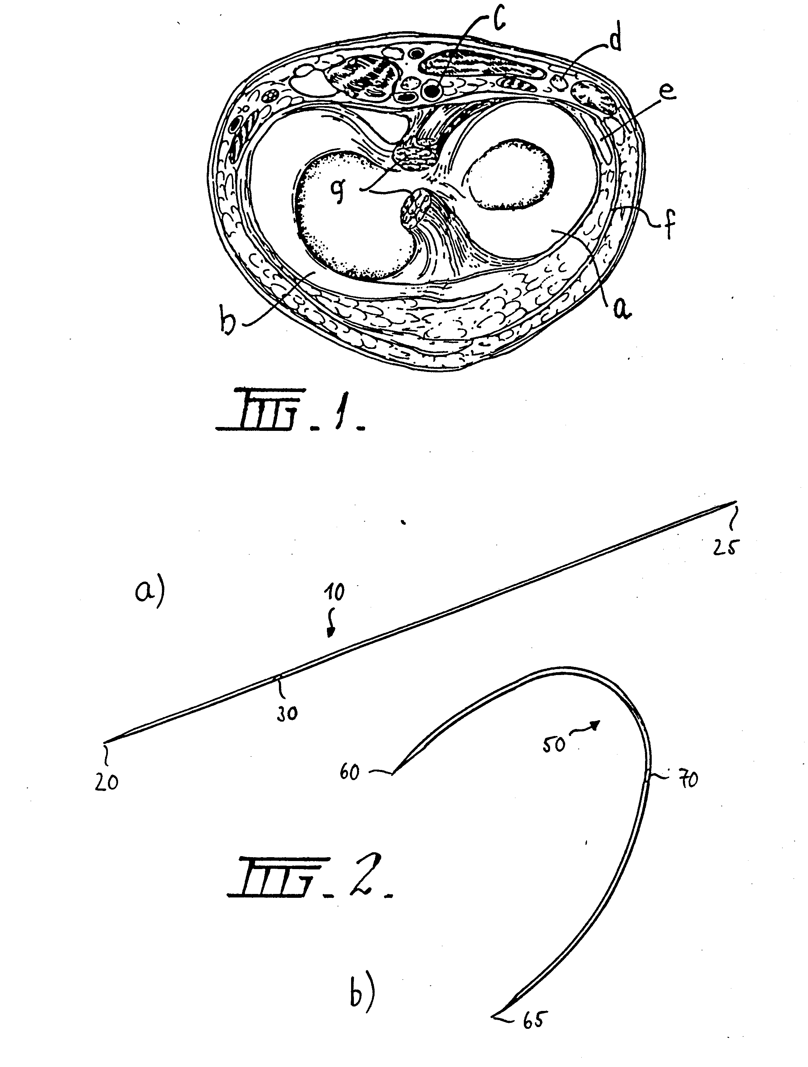

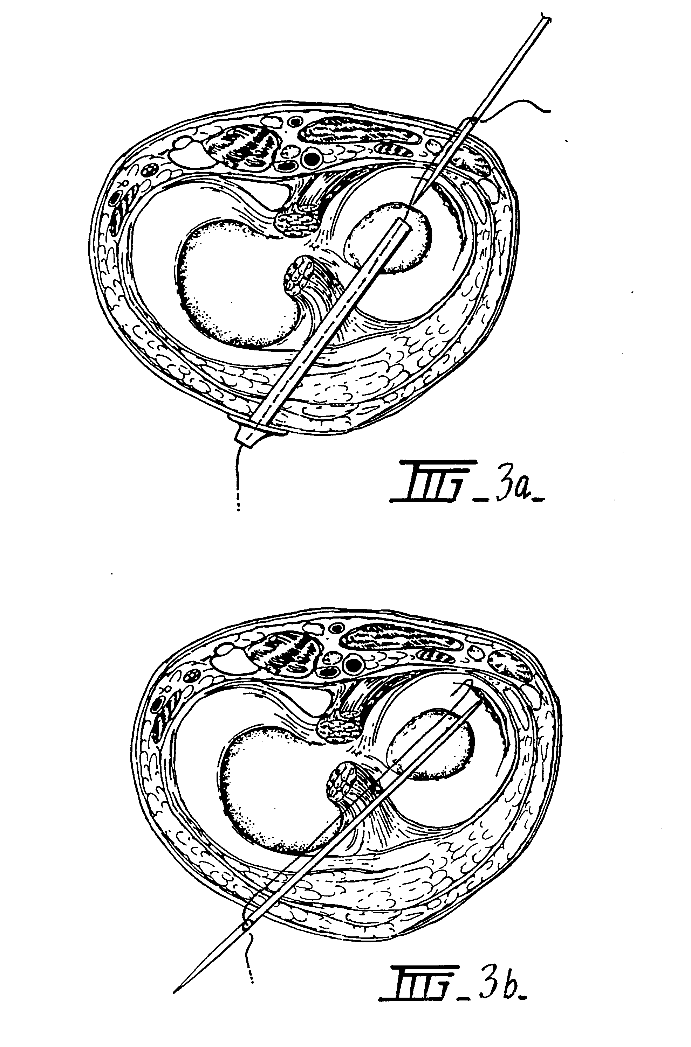

Tears in the meniscal cartilage of the knee joint are among the most common injuries presenting to orthopaedic surgeons. They are particularly common among sportsmen and women, especially footballers. As shown in Figure 1, in each knee there are two meniscal cartilages, the lateral meniscus (a) and the medial meniscus (b). Both of these menisci are vulnerable to tearing or rupture caused by torsional stress. Such meniscal lesions are frequently associated with injuries to the cruciate ligaments (Figure 1g).

Although at one time total or partial meniscectomy was the only available treatment for injuries of this type, it has become recognized that removal of the meniscus leads in many cases to subsequent degenerative arthritis of the knee joint (Jackson, J.P.: Brit. Med. J. (1968) 2 525-527). Although initial attempts at surgical conservation of the meniscus necessitated open surgery of the knee joint (Hamberg, P., J. Gillquist, and J. Lysholm: J. Bone and Joint Surgery (1983) 65A, 193-197), with consequent risk of infection and prolonged recovery time, more recently it has been shown that it is possible to perform the repair using an arthroscopic technique (Barber, F.A. and R.G. Stone: J. Bone and Joint Surgery (1985) 67B 39-41), employing two needles carrying a single suture thread.

Such operations are now commonly performed. Special needle holders are used (Barber and Stone, op. cit) or alternatively specially designed single or double channeled cannulas (Crane, L.: Clancy, W.G. J. and B.K. Graf: technical papers supplied with products) may be used. "Meniscal Stitcher" instruments are commercially available, for example the Acufex "Big Bend" stitcher and meniscal stitcher based on the second and third references cited in this paragraph ("Acufex" is a trade mark of Acufex Microsurgical, Inc., Massachusetts, U.S.A.).

All of the above described techniques present two major disadvantages. Firstly, it is not possible to knot the sutures within the joint space, and so the knots must be placed either externally to the joint capsule, (Figure 1f) necessitating a skin incision and dissection down to the capsule, or outside the skin over a sponge and button. The latter may result in chafing and maceration of the skin.

Secondly, the sites of egress of the needle from the joint are limited by the necessity to avoid damage to structures such as the popliteal artery (Figure 1c) and the common peroneal nerve (Figure 1d).

I have now developed a surgical needle which overcomes the limitations presented by those heretofore known.

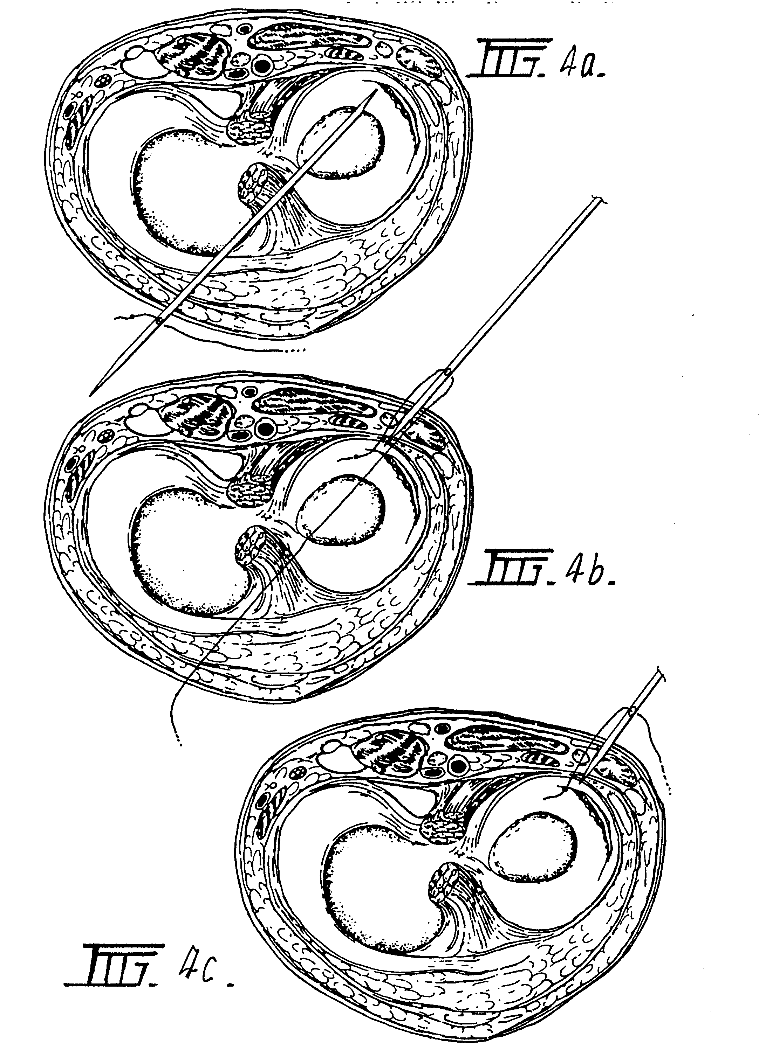

According to one preferred embodiment of the present invention there is provided a surgical needle 10 having two sharpened ends 20, 25 together with a centrally or eccentrically placed eye aperture 30.   This is illustrated in Figure 2a.

According to a second preferred embodiment, there is provided an atraumatic needle having two sharpened ends, in which the suture material is attached to the needle at a centrally or eccentrically placed site.

The material of the needle may be substantially rigid. Alternatively it may be partially flexible, so as to allow the needle to be used optionally in conjunction with a slightly curved cannula.

The ends of the needle may be of trocar type, or have a cutting edge.

Preferably the needle is made of surgical grade stainless steel.

A large range of sizes of needle may be used, depending upon the particular site of surgery, ranging from approximately 0.5 cm for microsurgical applications to 20-15 cm for orthopaedic surgery.

According to one particularly preferred embodiment, the needle is straight and has length in the range 15-25 cm, with the eye of the needle at approximately 2/3 to 5/7 of the length from one end. Preferably the length is 18 cm.

According to a second particularly preferred embodiment, the needle 50 is curved, having two sharpened ends 60, 65 and the eye aperture 70 placed midway along the length of the needle. This is illustrated in Figure 2b. A wide range of radii of curvature may be used from approximately 0.25 cm to infinity. The ends of the needle may be of trocar type or have a cutting edge.

The needle is suitable for use with any known type of suture material.

The present invention supplies a long-felt want with its capacity, simplicity and reliability, and it constitutes a significant advance in the procedures of tissue repair in a confined space.

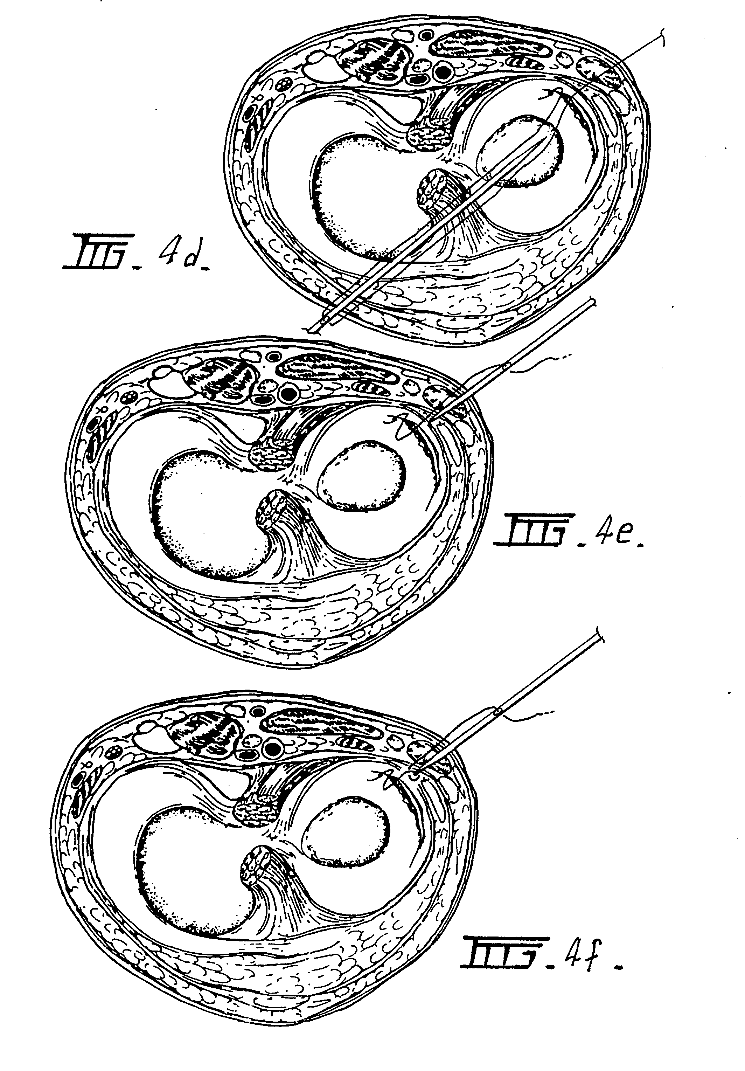

Figure 3 shows the advantage of the eccentrically placed eye aperture of one preferred embodiment of the invention. If the needle is passed into the knee joint through a cannula into the anterior medial aspect of the knee until the eye of the needle just leaves the skin on the posterior lateral side of the knee, the suture can be pulled through to the outside. However, the needle itself remains partly within the joint capsule (Figure 3a). The needle can then be passed back to the anterior medial side of the knee, thus reversing the manoeuvre, with the needle again remaining partly inside the joint capsule (Figure 3b).

Example 1    Repair of a Peripheral Tear in the Lateral Meniscus

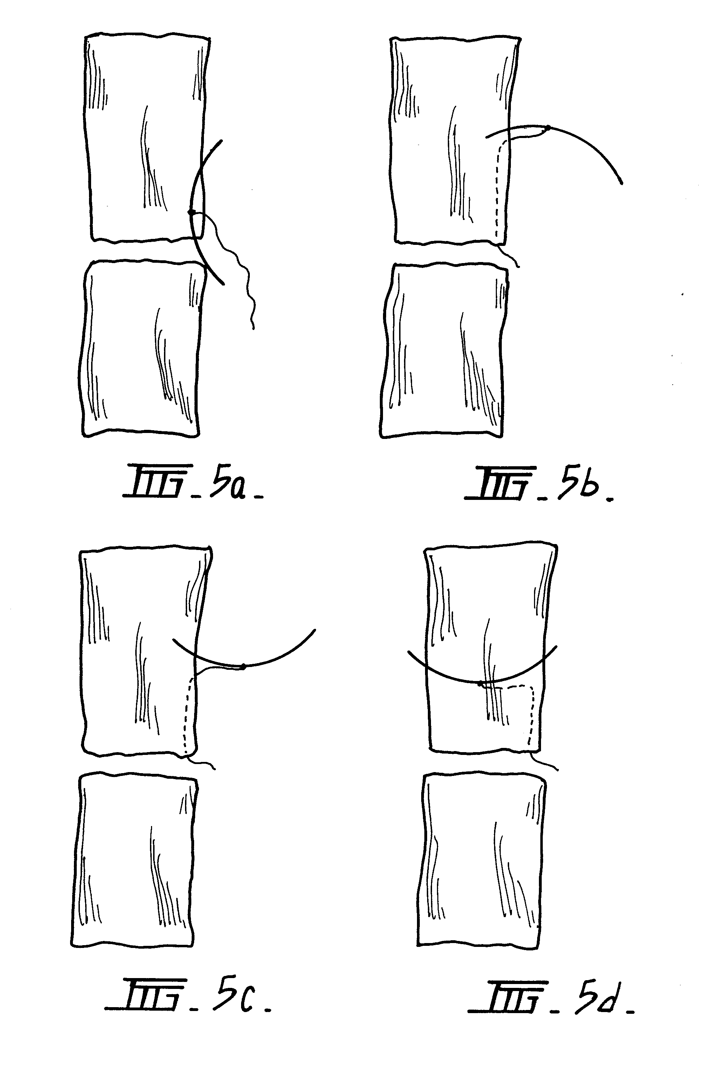

The straight needle, carrying a suture previously knotted at one end, is inserted via the end distal from the eye into the knee joint at the anterior medial aspect of the joint. Entry may be either directly through the skin, or more conveniently may be via the arthroscopic entry portal. Optionally a cannula may be used for greater accuracy in directing the needle. Under arthroscopic control using either direct vision or television monitor control, the tip of the needle is advanced until it reaches the medial side of the tear (Figure 4a).

The needle is then pushed through the meniscus and advanced so that the trailing tip of the needle remains inside the joint capsule, and the eye of the needle is just outside the skin of the knee on the lateral side, as shown in Figure 4b. The free end of the suture is pulled through to the exterior of the knee, as shown in Figure 4c. The needle is then pushed back through the meniscus in the reverse direction until the eye of the needle is just outside the skin and the tip of the longer arm of the needle is just adjacent to the meniscus, as shown in Figure 4d. Optionally the tip of the needle may remain within the meniscal tissue. The suture is pulled through to the exterior of the knee as before. The procedure is repeated as many times as required to repair the tear, as shown in Figure 4e.

Knots may be tied in the suture at intervals, either by passing the needle through a loop in the suture (Figure 4f), or, if the suture is not a monofilament material, by passing the needle through the suture material itself. Other methods of forming knots in the suture may also be used. The knots thus formed are within the joint itself.

A further advantage of the use of the needle of the present invention for such procedures is that if a structure such as a nerve, tendon or blood vessel is traversed by the needle, the suture may be pulled through without further damage to that structure.

Example 2    Repair of Ligaments or Tendons

Ligaments or tendons may be repaired by similar procedures to those described in Example 1, using either straight or curved needles according to the present invention. In the case of tendons in the hand, repair may be effected using the needle of the present invention without opening or suturing the tendon sheath.

Although the preferred embodiments of the invention have been described in detail it will be understood that many variations in the specific details thereof will be obvious to persons skilled in the art and the invention in its broader aspects contemplates all such variations falling within the more general disclosure hereinabove.

Example 3    Repair of Rotator Cuff Ligament in the Shoulder

The needle (either straight or curved) is passed through the skin either directly or via a cannula into the subacromial space.  Using either direct vision or orthroscopic control, the needle is directed at the rotator cuff ligament from one side of the tear to the other.  The tear is stitched as described for meniscus in Example 1.  Interrupted sutures may be used.

Example 4    Repair of Tendons

Tendons may be sutured preferably using the curved needle according to the invention, either in an open procedure or under arthroscopic control.

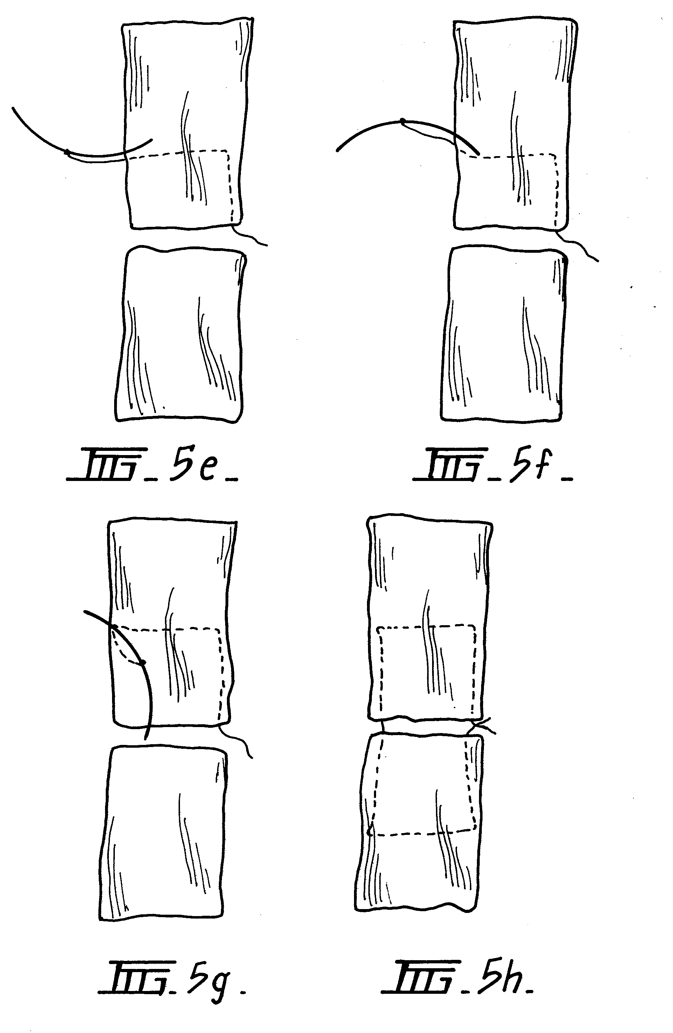

The needle is first passed through one cut end of the tendon, exiting from the side of the tendon (Figure 5a). With the trailing tip of the needle still inside the tendon, the direction of the needle is changed as shown in Figure 5c and d.  The dotted line represents the path of the suture. The needle is then passed through the tendon to the other side as shown in Figure 5d and e, so that the trailing tip of the needle again remains within the tendon.  The direction of the needle is changed again so that the leading tip is directed at the cut end of the tendon (Figure 5f and g).  The same procedure is repeated for the other cut end of the tendon.  By this procedure the suture material is entirely retained within the tendon tissue, and is invisible.  The final knot may be tied between the cut ends so that the knot is also hidden (Figure 5h).

0205811

CLAIMS

1.　　A surgical needle characterized by having two sharpened ends together with a centrally or eccentrically placed eye aperture in which the needle is 0.5 to 30 cm long and has a radius of curvature from 0.25 cm to infinity.

2.　　A surgical needle characterized by having two sharpened ends together with a suture attached at a centrally or eccentrically placed site, in which the needle is 0.5 to 30 cm long and has a radius of curvature from 0.25 cm to infinity.

3.　　A surgical needle according to Claim 1 or Claim 2 in which the material of the needle is substantially rigid.

4.　　A surgical needle according to Claim 1 or Claim 2 in which the material of the needle is partially flexible.

5.　　A surgical needle according to Claim 1 or Claim 2 adapted so as to be used in conjunction with a cannula.

6.　　A surgical needle according to any of the preceding claims in which the ends of the needle are of trocar type.

7.　　A surgical needle according to any of Claims 1 to 5 in which the ends of the needle have a cutting edge.

8.　　A surgical needle according to any of Claims 1 to 5 in which the ends of the needle are tapered.

9.　　A surgical needle according to any of the preceding claims in which the needle is made of surgical grade stainless steel.

10.　　A surgical needle according to any of the preceding claims, characterized in that the needle is straight and has length in the range 15-25 cm, with the eye of the needle at 2/3 to 5/7 of the length from one end.

11.　　A surgical needle according to Claim 10, in which the needle is 20 to 25 cm long and the eye of the needle is 2/3 of the length from one end.

12.　　A surgical needle according to Claim 10 in which the needle is 9 in long.

13.     A surgical needle according to any one of Claims 1 to 9, characterized in that the needle is curved, having two sharpened ends and the eye aperture is placed midway along the length of the needle.

14.     A surgical needle according to any of the preceding claims supplied with a pre-threaded suture.

0205811

1/6

FIG. 1.

a)

b)

FIG. 2.

0205811

FIG_3a_

FIG_3b_

FIG. 4a.

FIG. 4b.

FIG. 4c.

FIG. 4d.

FIG. 4e.

FIG. 4f.

FIG. 5a.

FIG. 5b.

FIG. 5c.

FIG. 5d.

_FIG_5e_

_FIG_5f_

_FIG_5g_

_FIG_5h_

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A- 867 420 (R. SCHEFFEL) <br><br> * Whole document * <br> --- | 1,2,10 -12 | A 61 B 17/00 |
| A | US-A-1 695 887 (Ch. DAVIS) <br><br> * Figures 1,2; page 1, lines 52-56,72-76,82-90; page 2, lines 29-35 * <br> --- | 1-4,7-9,14 | |
| A | US-A-2 833 284 (H. SPRINGER) <br> * Figures 1,3; column 2, lines 13-21; claim 1 * <br> --- | 5,13 | |
| A | US-A-1 835 287 (E. DONOVAN) <br><br> ----- | 6 | |

| | TECHNICAL FIELDS SEARCHED (Int. Cl.4) |
|---|---|
| | A 61 B |

The present search report has been drawn up for all claims

| Place of search | Date of completion of the search | Examiner |
|---|---|---|
| THE HAGUE | 22-09-1986 | SEDY, R. |

EPO Form 1503 03 82